# EUROPEAN PATENT SPECIFICATION

(11) **EP 3 194 961 B1**
(45) Date of publication and mention of the grant of the patent: **06.01.2021**
(21) Application number: 15842191.7
(22) Date of filing: 10.09.2015
(51) Int. Cl.: G01N 33/543, G01N 33/53, G01N 31/00, G01N 33/00, G01N 29/02, G01N 29/22, G01N 29/36

(54) **THIN FILM BULK ACOUSTIC RESONATOR WITH SIGNAL ENHANCEMENT**
DÜNNSCHICHT-VOLUMENSCHALLWELLENRESONATOR MIT SIGNALVERSTÄRKUNG
RÉSONATEUR ACOUSTIQUE À ONDE DE VOLUME EN FILM MINCE AVEC RÉHAUSSEMENT DE SIGNAL

(30) Priority: 15.09.2014 US 201462050589 P
(43) Date of publication of application: 26.07.2017
(73) Proprietor: QORVO US, INC., Greensboro, NC 27409 (US)
(72) Inventor: SALVATI, Michael, Minnetrista, MN 55364 (US); HARMON, Ian, Robert, Minneapolis, MN 55413 (US)
(74) Representative: J A Kemp LLP
(86) International application number: PCT/US2015/049387
(87) International publication number: WO 2016/044055

(56) References cited:
- WO-A1-2014/143680
- WO-A1-2016/044132
- WO-A2-2008/019693
- WO-A2-2014/012136
- CN-A- 1 566 933
- US-A- 4 999 284
- US-A- 5 932 953
- US-A1- 2006 125 489
- US-A1- 2012 196 384
- US-A1- 2013 224 732
- WINGQVIST ET AL: "Immunosensor utilizing a shear mode thin film bulk acoustic sensor", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, ELSEVIER BV, NL, vol. 127, no. 1, 5 October 2007 (2007-10-05), pages 248-252, XP022286108, ISSN: 0925-4005, DOI: 10.1016/J.SNB.2007.07.051
- WANG JINGJING ET AL: "Label-free immunosensor based on micromachined bulk acoustic resonator for the detection of trace pesticide residues", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 190, 8 September 2013 (2013-09-08), pages 378-383, XP028764422, ISSN: 0925-4005, DOI: 10.1016/J.SNB.2013.08.102
- CHEN DA ET AL: "A pure shear mode ZnO film resonator for the detection of organophosphorous pesticides", SENSORS AND ACTUATORS B: CHEMICAL: INTERNATIONAL JOURNAL DEVOTED TO RESEARCH AND DEVELOPMENT OF PHYSICAL AND CHEMICAL TRANSDUCERS, vol. 171, 26 June 2012 (2012-06-26), pages 1081-1086, XP028932227, ISSN: 0925-4005, DOI: 10.1016/J.SNB.2012.06.037

## Description

### FIELD

This disclosure relates to signal enhancement of thin film bulk acoustic resonators (TFBARs) through amplification element mediated mass loading.

### BACKGROUND

Piezoelectric devices such as thin film bulk acoustic resonators (TFBARs) and similar technologies like quartz crystal microbalances (QCM) have been employed as mass detectors for some time. One application of piezoelectric resonators is in detecting very small quantities of materials. Piezoelectric resonators used as sensors in such applications are sometimes called "micro-balances." A piezoelectric resonator is typically constructed as a thin, planar layer of crystalline or polycrystalline piezoelectric material sandwiched between two electrode layers. When used as a sensor, the resonator is exposed to the material being detected to allow the material to bind on a surface of the resonator.

One conventional way of detecting the amount of the material bound on the surface of a sensing resonator is to operate the resonator as an oscillator at its resonant frequency. As the material being detected binds on the resonator surface, the oscillation frequency of the resonator is reduced. The change in the oscillation frequency of the resonator, presumably caused by the binding of the material on the resonator surface, is measured and used to calculate the amount of the material bound on the resonator or the rate at which the material accumulates on the resonator surface.

The sensitivity of a piezoelectric resonator in air as a material sensor is theoretically proportional to the square of the resonance frequency. Thus, the sensitivities of material sensors based on the popular quartz crystal resonators are limited by their relatively low oscillating frequencies, which typically range from several MHz to about 100 MHz. The development of thin-film resonator (TFR) technology can potentially produce sensors with significantly improved sensitivities. A thin-film resonator is formed by depositing a thin film of piezoelectric material, such as AlN or ZnO, on a substrate. Due to the small thickness of the piezoelectric layer in a thin-film resonator, which is on the order of several microns, the resonant frequency of the thin-film resonator is on the order of 1 GHz. The high resonant frequencies and the corresponding high sensitivities make thin-film resonators useful for material sensing applications. However, mass sensitivity of even thin-film resonators may be limited for detection of certain analytes, such as biological analytes.

The use of piezoelectric resonator sensors in immunoassays has been described previously. In general piezoelectric based immunoassays, in which mass change is attributable to the immunological reaction between an antigen and an antibody, can in circumstances suffer from poor sensitivity and poor detection limit. Consequently, there is a need in the art for a piezoelectric-based specific binding assay in which the reaction between a molecular recognition component and its target analyte can be amplified to provide a more sensitive assay.

One such example is presented in U.S. Pat. No. 4,999,284 issued to Ward on 12 Mar. 1991, which discloses a method using a quartz crystal microbalance assay, in which the binding of analyte to a surface on or near a quartz crystal microbalance (QCM) is detected by a conjugate that includes an enzyme. The enzyme is capable of catalyzing the conversion of a substrate to a product capable of accumulating on or reacting with a surface of the QCM leading to a mass change and, hence, a change in resonant frequency. Alternative piezoelectric sensors for use in assays to detect an analyte are well-documented, for example bulk acoustic sensors (CN 1,566,933), thin film bulk acoustic resonators (US 2006/0125489), and surface acoustic wave sensors (WO 2008/019693). Thin film bulk acoustic resonators may utilize signal enhancement methods such as amplification element mediated mass loading, as presented in WO 2014/143680. WO 2014/143680 takes 15 March 2013 as its priority date and was published on 18 September 2014. It is therefore regarded as state of the art pursuant to Article 54(3) EPC.

### SUMMARY

This disclosure describes signal amplification to enhance sensitivity of TFBAR operating at a high frequency.

The present invention provides a method and a system for detecting an analyte in a sample. The method comprises:
contacting an analyte and optionally a tag-linked analyte, a first recognition component, and a second recognition component linked with an amplification element to generate a complex comprising the first recognition component and the second recognition component linked with the amplification element,
   wherein the first recognition component is immobilized on a surface of a thin film bulk acoustic resonator (TFBAR) having a resonance frequency of 1.8 GHz or greater and is configured to selectively bind one or more of the analyte and optionally a tag-linked analyte bound to the second recognition component,
   wherein the second recognition component linked with the amplification element is configured to selectively bind the analyte and optionally the tag-linked analyte bound to the first recognition component; and
   wherein the second recognition component is conjugated to a first binding partner, the amplification element is conjugated to a second binding partner configured to selectively bind to the first binding partner, and wherein the amplification element is linked to the second recognition component via binding between the first and second binding partners;
contacting the amplification element linked with an amplification precursor under conditions to convert the amplification precursor into a molecule that adds mass at a surface of the TFBAR; and
measuring mass added at the surface of the TFBAR.

The added mass may result from deposition of the amplification molecule on the surface; binding of the amplification molecule to one or more of the analyte, the tag-linked analyte molecule, the first recognition component or the second recognition component linked with an amplification element; or the like.

The system comprises:
a thin film bulk acoustic resonator (TFBAR) comprising a surface on which a first recognition component is immobilized,
   the first recognition component being configured to selectively bind the analyte and optionally a tag-linked analyte to which a second recognition component is bound, the TFBAR having a resonance frequency of 1.8 GHz or greater;
one or more containers housing: an amplification precursor; optionally one or more of the tag and the analyte; and the second recognition component and an amplification element, the second recognition component being optionally linked with the amplification element, wherein the second recognition component optionally linked with the amplification element comprises the second recognition component conjugated to a first binding partner and the amplification element conjugated to a second binding partner that is configured to selectively bind to the first binding partner, wherein, in use, the amplification element is linked to the second recognition component through binding of the first and second binding partners;
a fluid path from the one or more containers to the surface of the TFBAR to which the first recognition component is bound;
actuation circuitry configured to drive the TFBAR in an oscillating motion;
measurement circuitry arranged to be coupled to the TFBAR and configured to measure one or more resonator output signals representing resonance characteristics of the oscillating motion of the sensing resonator; and
a controller operatively coupled with the actuation and measurement circuitry.

The analyte or the analyte and the tag-linked analyte molecule, the first recognition component and the second recognition component linked with the amplification element may be contacted in any suitable order. For example, the analyte or the analyte and the tag-linked analyte molecule may be contacted with the second recognition component linked with the amplification element prior to contact with the first recognition component immobilized on the surface of the TFBAR. By way of further example, the analyte or the analyte and the tag-linked analyte molecule may be contacted with the first recognition component prior to contact with the second recognition component linked with the amplification element. By way of yet another example, the analyte or the tag-linked analyte molecule, the first recognition component and the second recognition component linked with the amplification element may be contacted simultaneously.

The amplification element may be linked to the second recognition component at any suitable time. In some embodiments, the amplification element is linked to the second recognition component prior to contact with the analyte or the tag-linked analyte molecule. In some embodiments, the amplification element is linked to the secondary recognition component after the second recognition component is contacted with the analyte or the tag-linked analyte. In some embodiments, the amplification element is linked to the second recognition component by a covalent bond. In some embodiments, the amplification element and the second recognition component include moieties that bind with high affinity. By way of example, the secondary recognition component may be biotinylated, and the amplification element may be conjugated to avidin or streptavidin; or vice-versa.

The mass added to or bound to the surface of the TFBAR may be measured by any suitable process. In preferred embodiments, the mass is measured by: (i) coupling an input electrical signal to the TFBAR, the input electric signal having a frequency within a resonance band of the piezoelectric resonator, wherein the frequency is 1.8 GHz or greater (such as about 2 GHz or greater, about 2.2 GHz or greater, about 2.4 GHz or greater, about 2.5 GHz or greater, or from about 2 GHz to about 2.5 GHz); (ii) transmitting the input electrical signal through or across the TFBAR to generate an output electrical signal having a frequency; (iii) receiving the output electrical signal from the TFBAR; and (iv) determining a change in phase shift of the output electrical signal caused by deposition of the precipitate on the surface of the TFBAR, wherein the change in phase shift serves as a measure of the mass added at the surface of the TFBAR

One or more embodiments of the systems or methods described herein provide one or more advantages over prior systems or methods for detecting small quantities of an analyte. As described herein, at higher frequencies larger TFBAR signal amplification was surprisingly observed with amplification element-mediated mass loading than at lower frequencies. Accordingly, advantages of higher frequencies appear to be even further enhanced when employed in combination with signal amplification. This and other advantages will be readily understood by those of skill in the art from the following detailed description.

### BRIEF DESCRIPTION OF THE DRAWINGS

**FIGS. 1A-1C** are schematic diagrams illustrating the operational principles of embodiments of thin film bulk acoustic resonator (TFBAR) sensing devices.
**FIG. 2** is a schematic diagram showing components of a TFBAR system for detecting an analyte.
**FIGS. 3A-D** are schematic drawings illustrating an embodiment of signal amplification on a surface of a thin film resonator (TFR).
**FIGS. 4A-D** are schematic drawings illustrating an embodiment of signal amplification on a surface of a TFR.
**FIGS. 5A-B** are schematic diagrams of an embodiment of a first binding partner bound to a surface of a TFR **(5A)** and a recognition component bound to a second binding partner, which is bound to the first binding partner **(5B).**

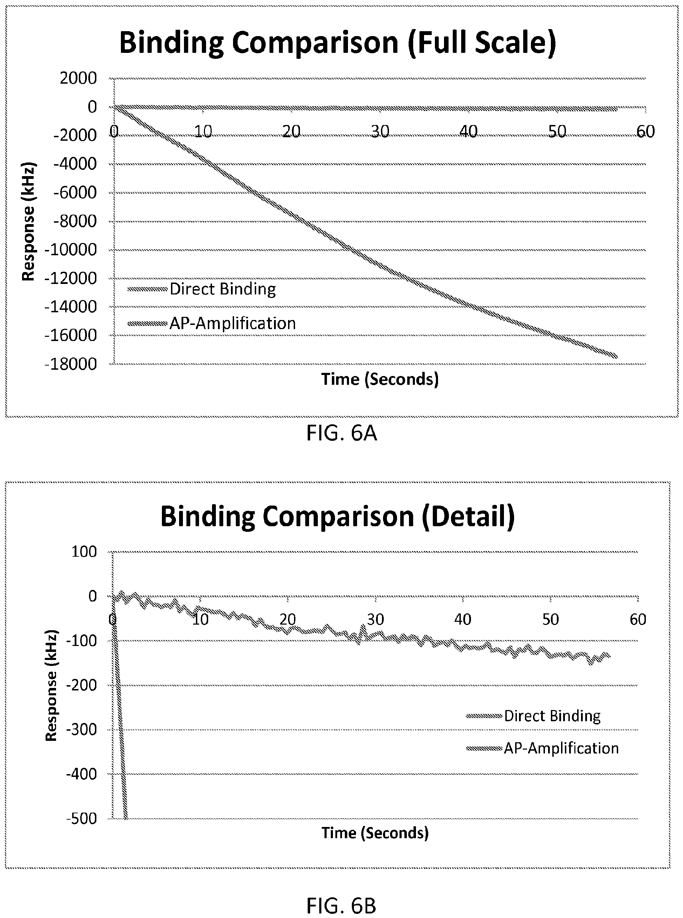

**FIG. 6A** is a plot of response over time of direct analyte binding and enzyme-amplified analyte binding on an embodiment of a TFBAR.
**FIG. 6B** is a plot showing details of a portion of the plot presented in **FIG. 4A****.**
**FIG. 7** is a schematic drawing illustrating an embodiment of various polynucleotide components bound to a surface of a TFR as described in EXAMPLE 2.

The schematic drawings are not necessarily to scale. Like numbers used in the figures refer to like components, steps and the like. However, it will be understood that the use of a number to refer to a component in a given figure is not intended to limit the component in another figure labeled with the same number. In addition, the use of different numbers to refer to components is not intended to indicate that the different numbered components cannot be the same or similar.

### DETAILED DESCRIPTION

This disclosure generally relates to methods, systems for detecting an analyte, as well as sensors and devices suitable for use in said methods and systems. The methods, devices, sensors and systems use a thin film bulk acoustic resonator (TFBAR) that measures a change in frequency or phase of the resonator caused by the binding of the analyte on a surface of the resonator. The binding signal is enhanced through amplification element-mediated mass loading. An input electrical signal having a phase and having a frequency within a resonance band of the piezoelectric resonator, which is 1.8 GHz or greater is coupled to and transmitted through the resonator to generate an output electrical signal which is frequency-shifted or phase-shifted from the input signal due to binding, deposition, etc. of material being detected on the resonator surface and amplification due to the amplification element-mediated mass loading. The output electrical signal received from the piezoelectric resonator is analyzed to determine the change in frequency or phase caused by the binding of analyte and amplification element-mediated mass deposition on the resonator surface. The measured change in frequency or phase provides quantitative information regarding the analyte (or tag-linked analyte molecule) bound to the resonator surface.

### Sensors, Devices and Systems

The sensors disclosed herein include at least one thin film resonator sensor, such as a thin film bulk acoustic resonator (TFBAR) sensor. A TFBAR sensor includes a piezoelectric layer, or piezoelectric substrate, and input and output transducer. TFBAR sensors are small sensors making the technology suitable for use in handheld devices. Accordingly, a handheld device for detecting target analytes comprising a sensor described herein is contemplated.

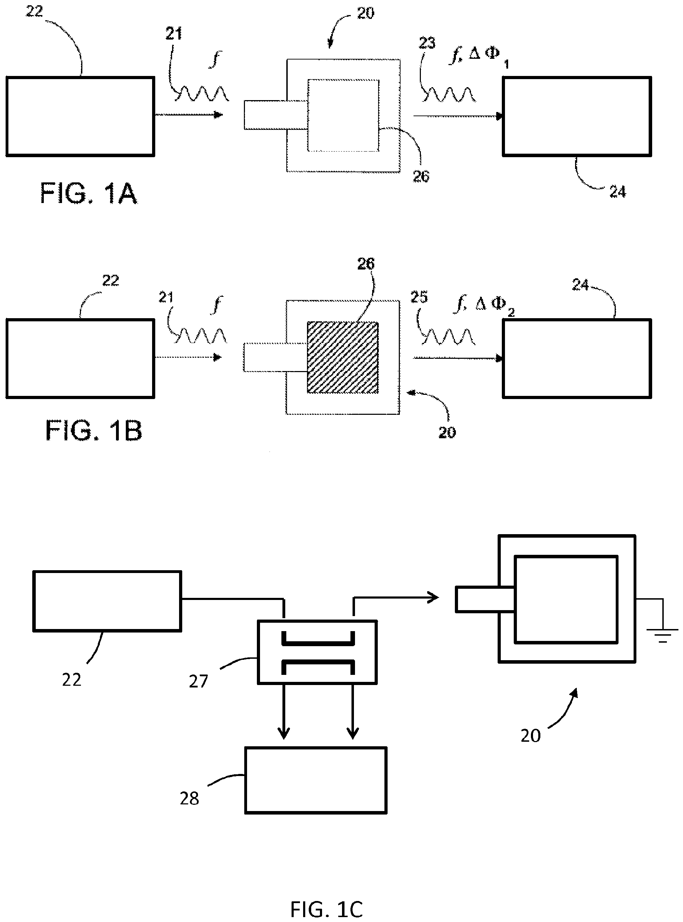

Turning now to the drawings with reference to **FIGS. 1A** and **1B****,** general operating principles of an embodiment of a bulk-acoustic wave piezoelectric resonator **20** used as a sensor to detect an analyte are shown. The resonator **20** typically includes a planar layer of piezoelectric material bounded on opposite sides by two respective metal layers which form the electrodes of the resonator. The two surfaces of the resonator are free to undergo vibrational movement when the resonator is driven by a signal within the resonance band of the resonator. When the resonator is used as a sensor, at least one of its surfaces is adapted to provide binding sites for the material being detected. The binding of the material on the surface of the resonator alters the resonant characteristics of the resonator, and the changes in the resonant characteristics are detected and interpreted to provide quantitative information regarding the material being detected.

By way of example, such quantitative information may be obtained by detecting a change in the insertion or reflection coefficient phase shift of the resonator caused by the binding of the material being detected on the surface of the resonator. Such sensors differ from those that operate the resonator as an oscillator and monitor changes in the oscillation frequency. Rather such sensors insert the resonator in the path of a signal of a pre-selected frequency and monitor the variation of the insertion or reflection coefficient phase shift caused by the binding of the material being detected on the resonator surface. Of course, sensors that monitor changes in oscillation frequency may also be employed in accordance with signal amplification described herein.

In more detail, **FIG. 1A** shows the resonator **20** before the material being detected is bound to its surface **26.** The depicted resonator **20** is electrically coupled to a signal source **22,** which provides an input electrical signal **21** having a frequency **f** within the resonance band of the resonator. The input electrical signal is coupled to the resonator **20** and transmitted through the resonator to provide an output electrical signal **23.** In the depicted embodiment, the output electrical signal **23** is at the same frequency as the input signal **21,** but differs in phase from the input signal by a phase shift **ΔΦ₁**, which depends on the piezoelectric properties and physical dimensions of the resonator. The output signal **23** is coupled to a phase detector **24** which provides a phase signal related to the insertion phase shift.

**FIG. 1B** shows the sensing resonator **20** with the material being detected bound on its surface **26.** The same input signal is coupled to the resonator **20.** Because the resonant characteristics of the resonator are altered by the binding of the material as a perturbation, the insertion phase shift of the output signal **25** is changed to **ΔΦ₂**. The change in insertion phase shift caused by the binding of the material is detected by the phase detector **24.** The measured phase shift change is related to the amount of the material bound on the surface of the resonator.

**FIG. 1C** shows an alternative to measuring the insertion phase of the resonator. A directional coupler **27** is added between the signal source **22** and the resonator **20** with the opposite electrode grounded. A phase detector **28** is configured to measure the phase shift of the reflection coefficient as a result of material binding to the resonator surface.

Other TFBAR phase-shift sensors that may be employed with the signal amplification described herein include those described in, for example, U.S. Patent No. 8,409,875 entitled "RESONATOR OPERTING FREQUENCY OPTIMIZATION FOR PHASE-SHIFT DETECTION SENSORS,". For example, sensor apparatuses may include (i) a sensing resonator comprising binding sites for an analyte; (ii) actuation circuitry configured to drive the sensing resonator in an oscillating motion; (iii) measurement circuitry arranged to be coupled to the sensing resonator and configured to measure one or more resonator output signals representing resonance characteristics of the oscillating motion of the sensing resonator; and (iv) a controller operatively coupled with the actuation and measurement circuitry. The controller can be interfaced with data storage containing instructions that, when executed, cause the controller to adjust the frequency at which the actuation circuitry drives the sensing resonator to maintain a resonance point of the sensing resonator. Accordingly, sensing may be accomplished by actuating the TFBAR into an oscillating motion; measuring one or more resonator output signals representing resonance characteristics of the oscillating motion of the TFBAR; and adjusting the actuation frequency of the sensing resonator to maintain a resonance point of the TFBAR. The frequency at which the actuation circuitry drives the sensing resonator may be a frequency of maximum group delay.

Such phase detection approaches can be advantageously used with piezoelectric resonators of different resonant frequencies.

TFBARs for use with the methods and system described herein has resonance frequencies of 1.8 GHz or greater, such as about 2 GHz or greater, 2.2 GHz or greater, 2.5 GHz or greater, about 3 GHZ or greater, or about 5 GHZ or greater. Said TFBARS can provide enhanced sensitivity when used with amplification element-mediated mass loading, which is described in more detail below. The TFBARs may have resonance frequencies of from 2 GHz to about 2.5 GHz. Some of such frequencies are substantially higher than frequencies of previously described piezoelectric resonators.

The sensing resonators described herein are thin-film resonators. Thin film resonators comprise a thin layer of piezoelectric material deposited on a substrate, rather than using, for example, AT-cut quartz. The piezoelectric films typically have a thickness of less than about 5 micrometers, such as less than about 2 micrometers, and may have thicknesses of less than about 100 nanometers. Thin-film resonators are generally preferred because of their high resonance frequencies and the theoretically higher sensitivities. Depending on the applications, a thin-film resonator used as the sensing element may be formed to support either longitudinal or shear bulk-acoustic wave resonant modes. Preferably, the sensing element is formed to support shear bulk-acoustic wave resonant modes, as they are more suitable for use in a liquid sample.

Additional details regarding sensor devices and systems that may employ TFRs are described in, for example, U.S. Patent No. 5,932,953 issued August 3, 1999 to Drees et al..

TFR sensors may be made in any suitable manner and of any suitable material. By way of example, a resonator may include a substrate such as a silicon wafer or sapphire, a Bragg mirror layer or other suitable acoustic isolation means, a bottom electrode, a piezoelectric material, and a top electrode.

Any suitable piezoelectric material may be used in a TFR. Examples of suitable piezoelectric substrates include lithium tantalate (LiTaO₃), lithium niobate (LiNbO₃), Zinc Oxide (ZnO), aluminum nitride (AlN), plumbum zirconate titanate (PZT) and the like.

Electrodes may be formed of any suitable material, such as aluminum, tungsten, gold, titanium, molybdenum, or the like. Electrodes may be deposited by vapor deposition or may be formed by any other suitable process.

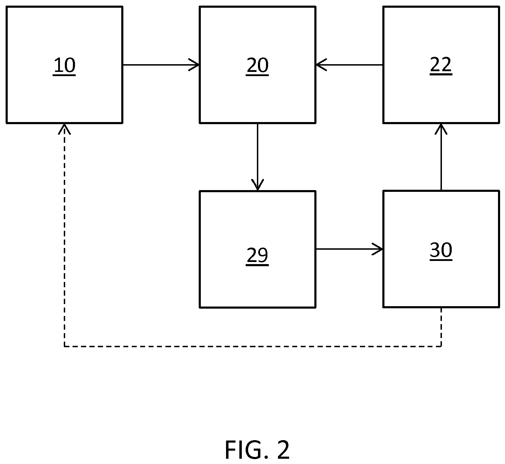

Any suitable system of the invention may employ a thin film resonator and amplification as described herein. By way of example and with reference to **FIG. 2****,** a system for detecting an analyte may include a container **10** (or more than one container), the thin film resonator **20,** actuation circuitry **22,** measurement circuitry **29,** and control electronics **30.** A fluid path couples the one or more containers **10** to the resonator **20.** The control electronics **30** are operably coupled to the actuation circuitry and the measurement circuitry. In embodiments, control electronics **30** are configured to modify the frequency at which the actuation circuitry **22** oscillates the resonator **20** based on input from the measurement circuitry **29.**

Still with reference to **FIG. 2****,** the container **10** (or more than one container) may house an amplification molecule, an amplification element-linked second recognition component or components thereof, and optionally one or more of a tag, an analyte molecule, and a first recognition component. Each of these reagents is described in more detail below. Control electronics **30** may control the flow of such reagents from container **10** to resonator **20;** e.g. via a pump, vacuum, or the like.

Any suitable control electronics **30** may be employed. For example, control electronics may include a processor, controller, memory, or the like. Memory may include computer-readable instructions that, when executed by processor or controller cause the device and control electronics to perform various functions attributed to device and control electronics described herein. Memory may include any volatile, non-volatile, magnetic, optical, or electrical media, such as a random access memory (RAM), read-only memory (ROM), non-volatile RAM (NVRAM), electrically-erasable programmable ROM (EEPROM), flash memory, or any other digital media. Control electronics **30** may include any one or more of a microprocessor, a controller, a digital signal processor (DSP), an application specific integrated circuit (ASIC), a field-programmable gate array (FPGA), or equivalent discrete or integrated logic circuitry. In some examples, control electronics **30** may include multiple components, such as any combination of one or more microprocessors, one or more controllers, one or more DSPs, one or more ASICs, or one or more FPGAs, as well as other discrete or integrated logic circuitry. The functions attributed to control electronics herein may be embodied as software, firmware, hardware or any combination thereof.

### Molecular Recognition and Signal Amplification

Molecular recognition of a sample comprising a significant background signal may be facilitated by amplification of the signal. The systems and methods described herein employ a second recognition component comprising an amplification element such as a linked enzyme. The TFBAR sensors, at the higher frequency ranges described herein, responded very efficiently to mass increase of the sensor surface due to precipitation of a substrate cleaved by an enzyme.

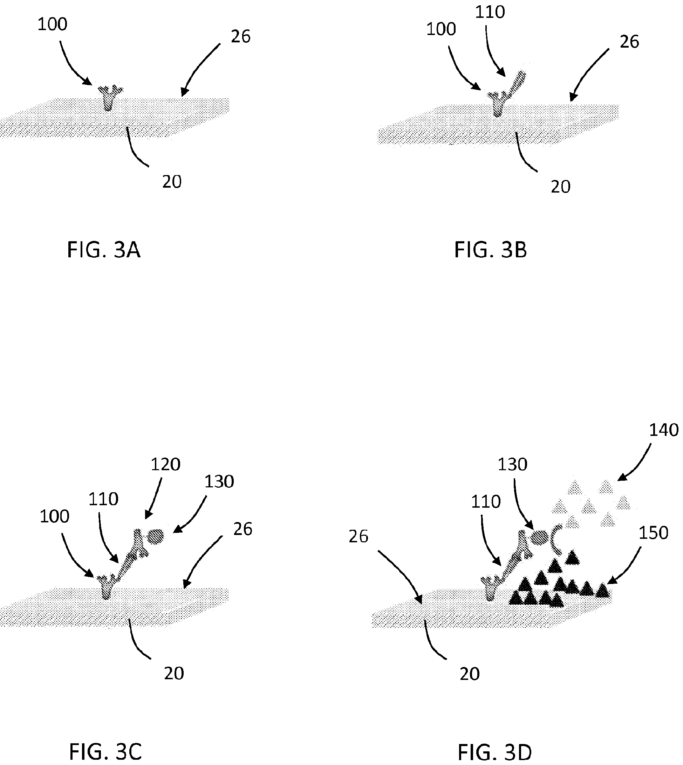

Referring now to **FIGS. 3A-D**, schematic drawings illustrating enzyme amplification on a TFBAR are shown. As depicted in **FIG. 3A****,** a molecular recognition component **100** configured to bind to an analyte is immobilized on a surface **26** of a resonator **20.** The resonator **20,** having immobilized molecular recognition component **100,** may be contacted with a composition comprising an analyte **110,** which may bind molecular recognition component **100** (see **FIG. 3B****).** The resonator **20,** having immobilized molecular recognition component **100** to which analyte **110** is bound, may be contacted with a composition comprising a second molecular recognition component **120** linked to an amplification element **130** such as an enzyme. The second molecular recognition component **120** is configured to bind to analyte **110** such that the second molecular recognition component **120** and linked amplification element **130** are immobilized relative to the surface **26** (see **FIG. 3C**). In the depicted embodiments, a soluble substrate **140** may be converted by amplification element **130** to an insoluble product **150,** which precipitates and accumulates on the surface **26** of the resonator **20,** thereby amplifying the mass signal as a function of amount or concentration of bound analyte **110** (see **FIG. 3D**).

It will be understood that the series of events depicted in **FIGS. 3A-3D** are shown for purposes of illustration and that any other suitable sequence of events may be employed. For example, the analyte **110** may be contacted with the second molecular recognition component **120** (and bound amplification element **130**) before the analyte (with bound second molecular recognition component) is contacted to the surface **26** of the resonator **20** relative to which the molecular recognition component **100** is immobilized. The substrate **140** may be present at the time the second molecular recognition component **120** - amplification element **130** is added or may be added later. In any case, washing may be performed prior to amplification.

Non-limiting examples of target analytes include nucleic acids, proteins, peptides, antibodies, enzymes, carbohydrates, chemical compounds, or infectious species such as bacteria, fungi, protozoa, viruses and the like. In certain applications, the target analyte is capable of binding more than one molecular recognition component.

Any suitable molecular recognition component (e.g., **100** in **FIG. 3**) may be bound to the surface of a resonator. The molecular recognition component preferably selectively binds to the analyte of interest. By way of example, the molecular recognition component may be selected from the group consisting of nucleic acids, nucleotide, nucleoside, nucleic acids analogues such as PNA and LNA molecules, proteins, peptides, antibodies including IgA, IgG, IgM, IgE, lectins, enzymes, enzymes cofactors, enzyme substrates, enzymes inhibitors, receptors, ligands, kinases, Protein A, Poly U, Poly A, Poly lysine, triazine dye, boronic acid, thiol, heparin, polysaccharides, coomassie blue, azure A, metal-binding peptides, sugar, carbohydrate, chelating agents, prokaryotic cells and eukaryotic cells.

Any suitable method for immobilizing a molecular recognition component on a surface of a TFBAR may be used. By way of example, a uniform coating of epoxy silane may be deposited on the sensor surface using a vapor deposition process. Test and reference molecular recognition components, such as antibodies, may then be deposited onto the test and reference resonators using, for example, piezo based nanodispensing technology. Primary amines on the antibodies react with the epoxide groups covalently binding the antibody to the sensor surface. By way of further example, a thiol group, if present, on the molecular recognition component may bind to a surface of the TFBAR. The surface of the TFBAR may be modified, as appropriate or necessary, to permit binding of the molecular recognition component.

Any suitable molecular recognition components, such as those described above, may be used as the second molecular recognition component (e.g., **120** in **FIG. 3**). The second molecular recognition component may be linked to any suitable amplification element, such as an enzyme. Preferably, the second molecular recognition component is an antibody and the amplification element is an enzyme.

Any suitable amplification element may be linked to the second molecular recognition component. The amplification element may be an activatable polymerization initiator, such as a photoinitiator, a chemical initiator, or a thermoinitiator. The polymerization initiator may be activated in the presence of one or more monomers to cause a polymer to graft from the second molecular recognition component. The amplification element may be an enzyme. The enzyme may be capable of converting a substrate that is soluble in the assay environment to an insoluble product that precipitates on the surface of the sensor. Examples of suitable enzymes include alkaline phosphatase (ALP), horse radish peroxidase (HRP), beta galactosidase, and glucose oxidase.

Examples of enzyme/substrate systems that are capable of producing an insoluble product which is capable of accumulating on a surface of a TFBAR include alkaline phosphatase and 5-bromo-4-chloro-3-indolylphosphate/nitro-blue tetrazolium chloride (BCIP/NBT). The enzymatically catalyzed hydrolysis of BCIP produces an insoluble dimer, which may precipitate on the surface of the sensors. Other analogous substrates having the phosphate moiety replaced with such hydrolytically cleavable functionalities as galactose, glucose, fatty acids, fatty acid esters and amino acids can be used with their complementary enzymes. Other enzyme/substrate systems include peroxidase enzymes, for example horse radish peroxidase (HRP) or myeloperoxidase, and one of the following: benzidene, benzidene dihydrochloride, diaminobenzidene, o-tolidene, o-dianisidine and tetramethyl-benzidene, carbazoles, particularly 3-amino-9-ethylcarbazole, and various phenolic compounds all of which have been reported to form precipitates upon reaction with peroxidases. Also, oxidases such as alphahydroxy acid oxidase, aldehyde oxidase, glucose oxidase, L-amino acid oxidase and xanthine oxidase can be used with oxidizable substrate systems such as a phenazine methosulfate-nitriblue tetrazolium mixture.

It will be understood that any type of competition assay may be employed. It will be further understood that the analyte may be modified to include a tag recognizable by the first or second recognition complex, such as a streptavidin tag; biotin tag; a chitin binding protein tag; a maltose binding protein tag; a glutathione-S-transferase tag; a poly(His) tag; an epitope tag such as a Myc tag, a HA tag, or a V5 tag; or the like. It will be further understood that the tag-linked analyte may include a variant or derivative of the analyte. The variant or derivative is a variant or derivative that is selectively recognizable by the first or second molecular recognition component that is configured to recognize the analyte. In some situations, it may be desirable that the variant or derivative analyte have an affinity for the first or second molecular recognition component that is different than the affinity of the non-tag-linked analyte. The variant or derivative of the analyte may be a variant or derivative that allows for ease of manufacture of the tag-linked analyte. For example, the tag-linked analyte may comprise a recombinant polypeptide, etc.

When competition assays employing tag-linked analyte molecules are performed, the tag-linked analyte molecule, rather than or in addition to the analyte, may bind a first molecular recognition component immobilized on a surface of a resonator.

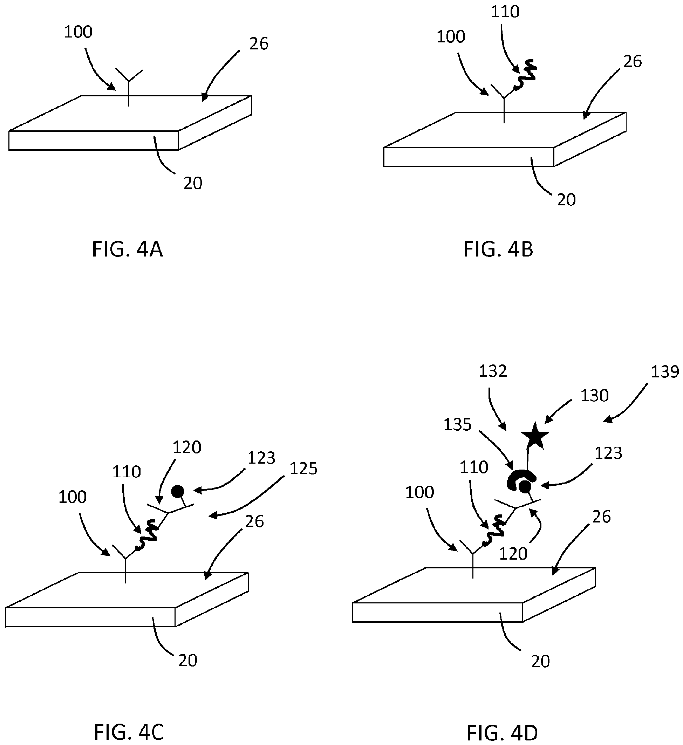

Referring now to **FIGS. 4****,** an embodiment of a signal amplification assay is depicted. Many of the components of **FIG. 4** are the same or similar to the components depicted in **FIG. 3****.** If a particular element is not specifically discussed with regard to **FIG. 4****,** reference is made to the numbered element in **FIG. 3** above. A signal amplification element **130** may be linked to second recognition component **120** at any suitable time. In some embodiments (not depicted in **FIG. 4**), the signal amplification element **130** is linked to the second recognition component **120** prior to contact with the analyte **110** or the tag-linked analyte molecule. In some of such embodiments, the signal amplification element **130** is covalently bound to the second recognition component **120.**

In some embodiments (e.g., as depicted in **FIGS. 4C-D**), signal amplification element **130** is linked to secondary recognition component **120** after second recognition component **120** is contacted with the analyte **110** or the tag-linked analyte. By way of example, second recognition component **120** can include first binding partner **123** configured to selectively bind second binding partner of signal amplification element **130.** First binding partner **123** is preferably covalently bound to second recognition component **120.** Second binding partner **135** is preferably covalently bound to signal amplification element **130.** First **123** and second **135** binding partners preferably bind with high affinity.

Any suitable combination of first **123** and second **135** binding partners may be employed. By way of example, secondary recognition component **120** can be biotinylated, and signal amplification element **130** may be conjugated to streptavidin; or vice-versa. By way of further example, one of first and second binding partners can be a polyhistidine(His) tag, and the other of first and second binding partners can be, for example, a nickel or copper chelator, such as iminodiacetic acid (Ni-IDA) and nitrilotriacetic acid (Ni-NTA) for nickel and carboxylmethylaspartate (Co-CMA) for cobalt, which the poly(His) tag can bind with micromolar affinity. Generally nickel-based resins have higher binding capacity, while cobalt-based resins offer the highest purity. By way of yet another example, one of first and second binding partners can be a glutathione-S-transferase (GST) tag, and the other of first and second binding partners can be glutathione. For yet another example, one of first and second binding partners can be a maltose binding protein tag, and the other of first and second binding partners can be amylose or maltose. As another example, one of first and second binding partners can be a chitin binding protein tag, and the other of first and second binding partners can be chitin. It will be understood that above-presented binding partners are merely examples of high affinity binding partners that may be conjugated to a second recognition component or a signal amplification element and that other binding partners are contemplated herein.

Binding partners may be conjugated to second recognition component or signal amplification element through any suitable technique. For example, chemical conjugation or recombinant techniques may be employed to link a binding partner to second recognition component or signal amplification element, as appropriate. Such techniques are well known to those of skill in the art. For example, a heterobifunctional cross linker utilizing NHS-ester and maleimide functional groups may be employed as known to those of skill in the art.

It will be understood that, if signal amplification element and second recognition component include complementary binding partners, the signal amplification element may be linked to second recognition component via binding partners at any suitable time. For example and as shown in **FIGS. 4C-D**, signal amplification element **130** may be linked to second recognition component **120** after second recognition component **120** is contacted with analyte **110** or tag-linked analyte. In some embodiments, signal amplification element **130** containing second binding partner **135** is contacted with second recognition component **120** containing first binding partner **123** before second recognition component **120** is contacted with analyte **110** or tag-linked analyte or at the same time as second recognition component **120** is introduced to resonator.

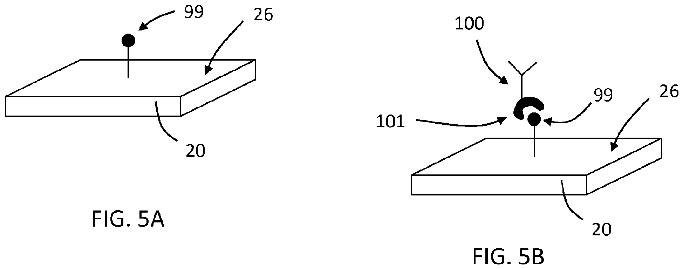

Referring now to **FIGS. 5A-B**, a first molecular recognition component **100** may be bound to a surface **26** of a TFBAR **20** via one or more intermediate. For example, a first binding partner **99** may be bound to the surface **26** and the first molecular recognition component **100** may include a second binding partner **101** configured to selectively bind to the first binding partner **99.** The binding partners **99, 101** may be binding partners as described above (e.g., with regard to **FIG.4****).** First recognition component **100** may be bound to surface **26** via binding partners **99, 101** at any suitable time, such as before the sensor **20** is incorporated into a system or after the sensor **20** is incorporated into the system. For example, first recognition component **100** may be bound to surface **26** via binding partners **99, 101** as a first step of, or during, an analyte detection assay.

### Amplification element-mediated mass loading/signal amplification with TFBARs

It has been noted that, as resonance frequency increases, sensitivity for mass detection should also increase. However, this is not always observed in practice. In theory, a TFBAR having a resonance frequency of about 2.2 GHz should afford sufficient sensitivity to detect low concentrations of analytes without the use of signal amplification/mass-loading as described herein. However, the inventors found that even with such high resonance frequencies, TFBAR sensors were not sufficiently sensitive to detect low levels of analyte. However, by utilizing the amplification/mass-loading techniques described herein, more of the theoretical gains in sensitivity offered by operating at higher frequencies can be realized.

Susceptibility to noise is related to signal propagation discussed theoretically above. At higher frequencies the signal propagates shorter distances, thus creating a proximity filter. That is, you only measure what is in proximity to the surface. However, what constitutes proximity will change with frequency and can have important practical ramifications with regard to susceptibility to background noise. Operation at higher frequencies with the mass loading not only results in enhanced signal sensitivity, it also results in lower susceptibility to noise. That can translate functionally to, for example, less stringent washing requirements because the second molecular recognition component linked with the amplification element, that is not bound to the surface of the resonator (e.g., via analyte bound to first molecular recognition component), should not add significant mass in proximity to the surface of the resonator. Furthermore, the washing requirements to obtain a stable baseline reading in negative sample were found to be much less stringent with higher frequency TFBARs, which may also be due to the shorter distance of signal propagation at higher frequency.

Surprisingly, it has been found that larger signal amplification is observed at higher frequencies than at lower frequencies. See, e.g. **Table 2** in the EXAMPLES below, where larger signal amplification was observed with enzyme mediated mass loading (relative to direct binding) at 2250 MHz compared with 900 MHz resonators. That larger levels of signal amplification can be obtained at higher frequencies was unexpected because the different resonators (900 MHz and 2250 MHz) were constructed to contain the same concentration or amount of first recognition component, assays used the same concentration and amount of analyte, and the same concentration and amount of enzyme-linked second molecular recognition component and substrate were used. Thus, in theory, the amount of amplification that actually occurs would be expected to be the same (the amount of product precipitated on the surface would be expected to be the same). However, a larger amount of amplification of signal was observed at higher frequencies.

### Use

The sensors, devices and systems described herein may be employed to detect an analyte in a sample. The sensors may find use in numerous chemical, environmental, food safety, or medial applications. By way of example, a sample to be tested may be, or may be derived from blood, serum, plasma, cerebrospinal fluid, saliva, urine, and the like. Other test compositions that are not fluid compositions may be dissolved or suspended in an appropriate solution or solvent for analysis.

### Definitions

All scientific and technical terms used herein have meanings commonly used in the art unless otherwise specified. The definitions provided herein are to facilitate understanding of certain terms used frequently herein and are not meant to limit the scope of the present disclosure.

As used in this specification and the appended claims, the singular forms "a", "an", and "the" encompass embodiments having plural referents, unless the content clearly dictates otherwise.

As used in this specification and the appended claims, the term "or" is generally employed in its sense including "and/or" unless the content clearly dictates otherwise. The term "and/or" means one or all of the listed elements or a combination of any two or more of the listed elements.

As used herein, "have", "having", "include", "including", "comprise", "comprising" or the like are used in their open ended sense, and generally mean "including, but not limited to". It will be understood that "consisting essentially of', "consisting of', and the like are subsumed in "comprising" and the like. As used herein, "consisting essentially of," as it relates to a composition, product, method or the like, means that the components of the composition, product, method or the like are limited to the enumerated components and any other components that do not materially affect the basic and novel characteristic(s) of the composition, product, method or the like.

The words "preferred" and "preferably" refer to embodiments of the invention that may afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the disclosure, including the claims.

Also herein, the recitations of numerical ranges by endpoints include all numbers subsumed within that range (e.g., 1 to 5 includes 1, 1.5, 2, 2.75, 3, 3.80, 4, 5, etc. or 10 or less includes 10, 9.4, 7.6, 5, 4.3, 2.9, 1.62, 0.3, etc.). Where a range of values is "up to" a particular value, that value is included within the range.

Any direction referred to herein, such as "top," "bottom," "left," "right," "upper," "lower," and other directions and orientations are described herein for clarity in reference to the figures and are not to be limiting of an actual device or system or use of the device or system. Devices or systems as described herein may be used in a number of directions and orientations.

"Binding event," as used herein, means the binding of a target analyte to a molecular recognition component immobilized in a surface of a sensor.

### EXAMPLES

The following non-limiting examples serve to describe more fully the manner of using the above described sensors, methods, devices and systems. It is understood that these examples in no way serve to limit the scope of this disclosure, but rather are presented for illustrative purposes. Embodiments falling outside the scope of the claims are included for reference purposes.

### EXAMPLE 1: Enzyme amplification proof of concept

Initial proof of concept studies were performed using an anti-bovine IgG assay and alkaline phosphatase (ALP) as the conjugated enzyme with BCIP/NBT as the precipitating substrate. Briefly, a goat anti-bovine and goat anti-rat antibodies were immobilized on the test and reference resonators by dispensing 350 µm spots using a piezo dispenser onto epoxy silane coated sensors with a resonate frequency of 2.2 GHz. Sensors were incubated overnight in a high humidity environment at 4°C. Sensors were blocked with fish skin gelatin prior to testing. The reference signal was then subtracted from the test signal and this delta signal used as the binding response. All testing performed with the sensors immersed in microtiter plates. Sample agitation achieved by using stirbars. The testing sequence was as follows, sensors were exposed to 1 µg/ml Bovine IgG for 60 seconds followed by a 30 second rinse and exposure to a rabbit anti-bovine IgG-alkaline phosphatase conjugate for 60 seconds. Sensors then rinsed 2 times for 30 seconds and exposed to BCIP/NBT substrate for 60 seconds. Sensors electrically connected to a network analyzer which was used to monitor the frequency shift of the devices. In this case, phase resulting in maximum group delay was tracked and change in input frequency to maintain the phase as mass changed was determined. A 50 MHz window around the resonate frequency was collect at a sampling rate of 2 samples per second for both the test and reference resonators. This data was post processed to determine the frequency shift as a function of time for both the test and reference resonators. The frequency shift observed from direct antigen binding was then compared to the signal observed in the enzyme substrate.

Results of this initial study are presented in **FIG. 4A****,** with **FIG. 4B** being a detailed view of a portion of the plot presented in **FIG. 4A****.** As shown, the ALP-enzyme amplification resulted in significant improvement in sensitivity relative to direct binding without addition of the substrate. As shown in **Table 1** below, over 100 fold amplification in response and slope was observed as a result of enzyme amplification.

**Table 1: Results of ALP-Amplification**

| | Direct Binding | Enzyme Amplification |
|---|---|---|
| Response (Integral) | -9,024 | -1,094,473 |
| Amplification (X) | 121 | |
| Slope | -3.24 | -374.77 |
| Slope Amp | 115.71 | |

Similar studies were performed using anti-bovine IgG assay and horse radish peroxidase (HRP) as the conjugated enzyme. The precipitating substrate in this reaction was hydrogen peroxide and p-hydroxycinnamic acid (data not shown). Further analysis on the ALP enhancement with BCIP/NBT using an anti-rat IgG assay was also performed (data not shown). Subsequent development work with the ALP and BCIP/NBT system has included evaluation with a variety of other.

The benefits of precipitate amplification appear to be frequency dependent. Devices with operating frequencies of 2250 MHz and 850 MHz were coated with a Goat anti-Rat F(ab') fragment. The native sulfhydryl group on the reduced F(ab') was used to link to the gold resonator surface forming a dative sulfur gold bond. These sensors were then blocked with fish skin gelatin and tested in negative buffer sample or 1 µg/ml Rat IgG followed by incubation with a goat anti-Rat alkaline phosphatase conjugated antibody. Sensors were then rinsed two times and exposed to BCIP/NBT substrate. Data was reduced as previously described for both the direct binding event and the substrate amplification. Comparison of the 2250 MHz to 900 MHz TFBARs demonstrate a 2.5 fold increase in signal amplification with the higher frequency (2250 MHz) relative to the 850 MHz devices. Additionally, the level of background observed in negative sample was considerably higher in the 850 MHz devices. This was true when the data was analyzed as kHz/sec of response but the difference became even more dramatic when the results were converted to ppm/sec by dividing the frequency shift response by the frequency of operation. The addition of two extra wash steps prior to substrate exposure reduced the amount of background signal in the 850 MHz devices to levels comparable to those seen in the 2250 MHz devices (data not shown).

**Table 2: Comparison of amplification with 850 and 2250 MHz TFBAR**

| | | |
|---|---|---|
| Frequency (MHz) | 2250 | 850 |
| Background Signal in Negative (ppm/sec) | -0.86 | -23.11 |
| Direct Binding (kHz/sec) | -3.26 | -0.70 |
| Amplified signal (kHz/sec) | -233.7 | -22.3 |
| Amplification (X) | 71.7 | 31.9 |

### EXAMPLE 2: DNA Proof of Feasibility

For DNA binding to the sensors surface a 10 µM 5'-amine labeled 27-mer (complementary to the 3' end of the target oligonucleotide) and 10 µM of a missense 27-mer were both dissolved in 3X saline-sodium citrate (3X SSC) and spotted on epoxy silane functionalized sensors as the test and reference respectively (2150 MHz TFBAR).

A 125-mer oligonucleotide was used as a model target. The target oligonucleotide was mixed with 6 nM of a 3'biotin labeled 18-mer complementary to the 5' end of the 125-mer target in hybridization buffer (5X SSC, 10% formamide, 0.1% SDS) and reacted with the sensor surface for 4 minutes at 39C.

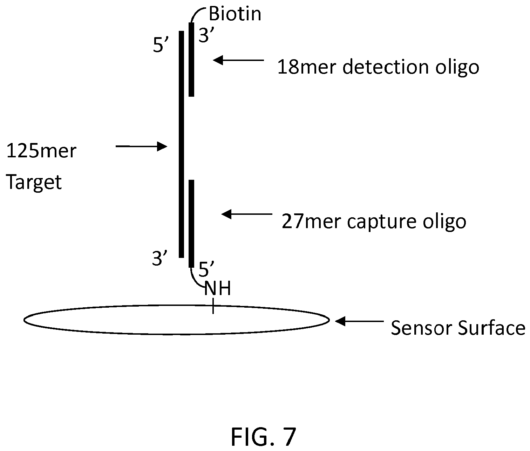

A schematic diagram of the 27-mer bound to the sensor surface and the target 125-mer, which is bound to the biotinylated 18-mer is shown in **FIG. 7****.**

Two wash steps were then performed (1X SSC with 0.01% SDS and HEPES buffered saline plus detergent) and the sensor was exposed to 2 µg/ml streptavidin-alkaline phosphatase conjugate purchased from Jackson Immuo (Part Number 016-050-084) for 2 minutes in HEPES buffer containing 1 mg/ml fish skin gelatin (FSG).

Two additional wash steps were performed with HEPES plus detergent and the sensor was exposed to the precipitating substrate NBT/BCIP purchased from Thermo (Part Number 34042). Frequency shift data was collected for 1.5 minutes at 39C and is presented below in **Table 3.** Estimated limit of detection using the zero response plus 3 standard deviations yielded a value of 0.6 pM.

**Table 3: Frequency Shift Data**

| DNA Target (pM) | Average Response (kHz/sec) |
|---|---|
| 0 | 0.93 |
| 0.8 | -0.48 |
| 4 | -2.93 |
| 40 | -38.15 |
| 400 | -111.61 |

### EXAMPLE 3: Interleukin-6 (IL-6) Two-Step Immunoassay

### Reagents:

Affinity purified goat anti IL-6 (R&D Systems Part Number AF-206-NA) was spotted onto epoxy silane activated sensors (2175 MHz TFBAR) generally as discussed above for the antibody in EXAMPLE 1.

Mouse monoclonal antibody against IL-6 (R&D Systems Part Number MAB206) was labeled with a 5X molar excess of sulfo-NHS-LC-Biotin (Thermo Scientific) according to the manufacturer's instructions. Excess unincorporated biotin reagent was removed by desalting.

Calibrator matrix was prepared by mixing 10% (v/v) chicken serum (Equitech, charcoal stripped, heat inactivated) with phosphate buffered saline (PBS) plus 0.1% sodium azide. Calibrators were prepared by diluting recombinant human IL-6 (R&D Systems Part Number 206-IL) in Calibrator matrix.

Streptavidin conjugated to alkaline phosphatase (SA-ALP) was purchased from Jackson Immuno (Part Number 016-050-084).

Wash buffer is Hepes buffered saline plus detergent.

Substrate is 1-Step NBT/BCIP purchased from Thermo Scientific (Part Number 34042).

### Assay:

Biotinylated mouse anti IL-6 was diluted to a working strength of 8.3 µg/mL in a Hepes buffer containing fish skin gelatin (Hepes/FSG).

SA-ALP was diluted to a working strength of 1 µg/mL in Hepes/FSG.

60 µL of biotinylated mouse anti IL-6 was mixed with 40 µL of calibrator and passed back and forth over the sensor for 16 minutes. The reaction mixture was then removed from the microfluidic channel and SA-ALP was passed back and forth over the sensor for 2 minutes. The sensor was then washed three times with Wash buffer followed by addition of BCIP/NBT substrate. The rate of change in resonance frequency during the substrate step was plotted with respect to IL-6 concentration. The results are shown in the **Table** 4 below including an estimate of the analytical sensitivity (zero response plus 3 standard deviations).

**Table 4: Frequency Shift for IL-6 Assay**

| IL6 | Response (kHz/sec) | | Calculated | | | | |
|---|---|---|---|---|---|---|---|
| (pg/mL) | Run | Avg | StDev | CV | (pg/mL) | Residuals | % Recovery |
| 0 | -2.28 | -2.12 | 0.23 | 11% | 0.83 | 0.83 | |
| | -1.96 | | | | <0.83 | NA | NA |
| 5 | -3.53 | -3.48 | 0.08 | 2% | 5.83 | 0.83 | 116.6 |
| | -3.42 | | | | 5.42 | 0.42 | 108.4 |
| 10 | -3.75 | -4.67 | 0.88 | 19% | 6.65 | -3.35 | 66.5 |
| | -4.75 | | | | 10.23 | 0.23 | 102.3 |
| | -5.51 | | | | 12.87 | 2.87 | 128.7 |
| 25 | -8.61 | -9.24 | 0.89 | 10% | 23.32 | -1.68 | 93.28 |
| | -9.87 | | | | 27.48 | 2.48 | 109.92 |
| 100 | -28.49 | -31.44 | 2.77 | 9% | 90.05 | -9.95 | 90.05 |
| | -33.99 | | | | 109.87 | 9.87 | 109.87 |
| | -31.85 | | | | 102.05 | 2.05 | 102.05 |
| **0+3SD** | **-2.82** | **analytical sensitivity estimate** | | | **3.11** | | |

## Claims

1. A method for detecting an analyte in a sample, comprising:
contacting an analyte (110) and optionally a tag-linked analyte, a first recognition component (100), and a second recognition component (120) linked with an amplification element (130) to generate a complex comprising the first recognition component (100) and the second recognition component (120) linked with the amplification element (130),
wherein the first recognition component (100) is immobilized on a surface (26) of a thin film bulk acoustic resonator (TFBAR) (20) having a resonance frequency of 1.8 GHz or greater and is configured to selectively bind one or more of the analyte (110) and optionally a tag-linked analyte bound to the second recognition component (120),
wherein the second recognition component (120) linked with the amplification element (130) is configured to selectively bind the analyte (110) and optionally the tag-linked analyte bound to the first recognition component (100); and
wherein the second recognition component (120) is conjugated to a first binding partner (123), the amplification element (130) is conjugated to a second binding partner (135) configured to selectively bind to the first binding partner (123), and wherein the amplification element (130) is linked to the second recognition component (120) via binding between the first and second binding partners (123 and 135);
contacting the amplification element (130) linked with an amplification precursor (140) under conditions to convert the amplification precursor (140) into a molecule (150) that adds mass at a surface (26) of the TFBAR (20); and
measuring mass added at the surface (26) of the TFBAR (20).

2. The method of claim 1, wherein the analyte (110) or the analyte and the tag-linked analyte are contacted with the second recognition component (120) linked with the amplification element (130) prior to contact with the first recognition component (100) immobilized on the surface (26) of the TFBAR (20).

3. The method of claim 1, wherein the analyte (110) or the analyte and the tag-linked analyte are contacted with the first recognition component (100) prior to contact with the second recognition component (120) linked with the amplification element (130).

4. The method of claim 1, wherein the analyte (110) or the tag-linked analyte, the first recognition component (100) and the second recognition component (120) linked with the amplification element (130) are contacted simultaneously.

5. The method of any one of claims 1-4, wherein measuring the mass added to or bound to the surface (26) of the TFBAR (20) comprises:
coupling an input electrical signal to the TFBAR (20), the input electric signal having a frequency within a resonance band of the piezoelectric resonator, wherein the frequency is 1.8 GHz or greater;
transmitting the input electrical signal through or across the TFBAR (20) to generate an output electrical signal having a frequency;
receiving the output electrical signal from the TFBAR (20); and
determining a change in phase shift of the output electrical signal caused by deposition of the precipitate on the surface (26) of the TFBAR (20).

6. The method of claim 5, wherein the change in phase shift is a change in insertion or reflection coefficient phase shift.

7. The method of any one of claims 1-4, wherein measuring the mass added to or bound to the surface (26) of the TFBAR (20) comprises:
actuating the TFBAR (20) into an oscillating motion at a frequency of about 1.8 GHz or greater;
measuring one or more resonator output signals representing resonance characteristics of the oscillating motion of the TFBAR (20); and
adjusting the actuation frequency of the sensing resonator to maintain a resonance point of the TFBAR (20).

8. The method of claim 7, wherein the resonance point of the TFBAR (20) is a point of maximum group delay.

9. The method of any one of claims 5-8, wherein the frequency is 2 GHz or greater.

10. The method of any one of claims 5-8, wherein the frequency is from 2 GHz to 2.5 GHz.

11. The method of any one of claims 1-10, wherein the amplification element (130) is an enzyme and the amplification precursor (140) is a substrate, and wherein the enzyme is configured to convert the substrate to a precipitate.

12. A system for detecting an analyte in a sample, comprising:
a thin film bulk acoustic resonator (TFBAR) (20) comprising a surface (26) on which a first recognition component (100) is immobilized,
the first recognition component (100) being configured to selectively bind the analyte (110) and optionally a tag-linked analyte to which a second recognition component (120) is bound, the TFBAR (20) having a resonance frequency of 1.8 GHz or greater;
one or more containers (10) housing: an amplification precursor (140); optionally one or more of the tag and the analyte (110); and the second recognition component (120) and an amplification element (130), the second recognition component (120) being optionally linked with the amplification element (130), wherein the second recognition component (120) optionally linked with the amplification element (130) comprises the second recognition component (120) conjugated to a first binding partner (123) and the amplification element (130) conjugated to a second binding partner (135) that is configured to selectively bind to the first binding partner (123), wherein, in use, the amplification element (130) is linked to the second recognition component (120) through binding of the first and second binding partners (123 and 135);
a fluid path from the one or more containers (10) to the surface (26) of the TFBAR (20) to which the first recognition component (100) is bound;
actuation circuitry (22) configured to drive the TFBAR (20) in an oscillating motion;
measurement circuitry (29) arranged to be coupled to the TFBAR (20) and configured to measure one or more resonator output signals representing resonance characteristics of the oscillating motion of the sensing resonator; and
a controller (30) operatively coupled with the actuation and measurement circuitry (22 and 29) .

## Patentansprüche

1. Verfahren zum Nachweis eines Analyten in einer Probe, das Folgendes umfasst:
Inkontaktbringen eines Analyten (110) und optional eines markierungsgebundenen Analyten, einer ersten Erkennungskomponente (100) und einer zweiten Erkennungskomponente (120), die mit einem Amplifikationselement (130) verbunden sind, um einen Komplex zu erzeugen, der die erste Erkennungskomponente (100) und die zweite Erkennungskomponente (120) umfasst, die mit dem Amplifikationselement (130) verbunden sind,
wobei die erste Erkennungskomponente (100) auf einer Oberfläche (26) eines akustischen Dünnschicht-Volumenresonators (TFBAR) (20) mit einer Resonanzfrequenz von 1,8 GHz oder mehr immobilisiert ist und dafür konfiguriert ist, selektiv einen oder mehrere der Analyten (110) und optional einen taggebundenen Analyten, der an die zweite Erkennungskomponente (120) gebunden ist, zu binden;
wobei die zweite Erkennungskomponente (120), die mit dem Amplifikationselement (130) verbunden ist, dafür konfiguriert ist, den Analyten (110) und optional den markengebundenen Analyten, der an die erste Erkennungskomponente (100) gebunden ist, selektiv zu binden; und
wobei die zweite Erkennungskomponente (120) an einen ersten Bindungspartner (123) konjugiert ist, das Amplifikationselement (130) an einen zweiten Bindungspartner (135) konjugiert ist, der dafür konfiguriert ist, selektiv an den ersten Bindungspartner (123) zu binden, und wobei das Amplifikationselement (130) über die Bindung zwischen dem ersten und dem zweiten Bindungspartner (123 und 135) mit der zweiten Erkennungskomponente (120) verbunden ist;
Inkontaktbringen des Amplifikationselements (130), das mit einem Amplifikationsvorläufer (140) verbunden ist, unter Bedingungen, um den Amplifikationsvorläufer (140) in ein Molekül (150) umzuwandeln, das Masse an einer Oberfläche (26) des TFBAR (20) hinzufügt; und
Messen der an der Oberfläche (26) des TFBAR (20) hinzugefügten Masse.

2. Verfahren nach Anspruch 1, wobei der Analyt (110) oder der Analyt und der markierungsgebundene Analyt mit der zweiten Erkennungskomponente (120), die mit dem Amplifikationselement (130) verbunden ist, vor dem Kontakt mit der ersten Erkennungskomponente (100), die auf der Oberfläche (26) des TFBAR (20) immobilisiert ist, in Kontakt gebracht werden.

3. Verfahren nach Anspruch 1, wobei der Analyt (110) oder der Analyt und der markierungsgebundene Analyt mit der ersten Erkennungskomponente (100) vor dem Kontakt mit der zweiten Erkennungskomponente (120), die mit dem Amplifikationselement (130) verbunden ist, in Kontakt gebracht werden.

4. Verfahren nach Anspruch 1, wobei der Analyt (110) oder der markierungsgebundene Analyt, die erste Erkennungskomponente (100) und die zweite Erkennungskomponente (120), die mit dem Amplifikationselement (130) verbunden sind, gleichzeitig kontaktiert werden.

5. Verfahren nach einem der Ansprüche 1 bis 4, wobei das Messen der Masse, die der Oberfläche (26) der TFBAR (20) hinzugefügt oder an diese gebunden wird, Folgendes umfasst:
Koppeln eines elektrischen Eingangssignals an den TFBAR (20), wobei das elektrische Eingangssignal eine Frequenz innerhalb eines Resonanzbandes des piezoelektrischen Resonators aufweist, wobei die Frequenz 1,8 GHz oder mehr beträgt;
Übertragen des elektrischen Eingangssignals durch oder über den TFBAR (20), um ein elektrisches Ausgangssignal mit einer Frequenz zu erzeugen;
Empfangen des elektrischen Ausgangssignals von dem TFBAR (20); und
Bestimmen einer Änderung der Phasenverschiebung des elektrischen Ausgangssignals, die durch Ablagerung des Niederschlags auf der Oberfläche (26) des TFBAR (20) verursacht wird.

6. Verfahren nach Anspruch 5, wobei die Änderung der Phasenverschiebung eine Änderung der Phasenverschiebung des Einfügungs- oder Reflexionskoeffizienten ist.

7. Verfahren nach einem der Ansprüche 1 bis 4, wobei das Messen der Masse, die der Oberfläche (26) der TFBAR (20) hinzugefügt oder an diese gebunden wird, Folgendes umfasst:
Betätigen des TFBAR (20) in eine oszillierende Bewegung mit einer Frequenz von etwa 1,8 GHz oder mehr;
Messen eines oder mehrerer Resonatorausgangssignale, die Resonanzeigenschaften der Schwingbewegung des TFBAR (20) darstellen; und
Einstellen der Betätigungsfrequenz des Erfassungsresonators, um einen Resonanzpunkt des TFBAR (20) aufrechtzuerhalten.

8. Verfahren nach Anspruch 7, wobei der Resonanzpunkt des TFBAR (20) ein Punkt maximaler Gruppenverzögerung ist.

9. Verfahren nach einem der Ansprüche 5 bis 8, wobei die Frequenz 2 GHz oder mehr beträgt.

10. Verfahren nach einem der Ansprüche 5 bis 8, wobei die Frequenz 2 GHz bis 2,5 GHz beträgt.

11. Verfahren nach einem der Ansprüche 1 bis 10, wobei das Amplifikationselement (130) ein Enzym und der Amplifikationsvorläufer (140) ein Substrat ist und wobei das Enzym dafür konfiguriert ist, das Substrat in einen Niederschlag umzuwandeln.

12. System zum Nachweis eines Analyten in einer Probe, das Folgendes umfasst:
einen akustischen Dünnschicht-Volumenresonator (TFBAR) (20) mit einer Oberfläche (26), auf der eine erste Erkennungskomponente (100) immobilisiert ist,
wobei die erste Erkennungskomponente (100) dafür konfiguriert ist, den Analyten (110) und optional einen markierungsgebundenen Analyten, an den eine zweite Erkennungskomponente (120) gebunden ist, selektiv zu binden, wobei der TFBAR (20) eine Resonanzfrequenz von 1,8 GHz oder mehr aufweist;
einen oder mehrere Behälter (10), in denen Folgendes untergebracht ist: ein Amplifikationsvorläufer (140); optional die Markierung und/oder der Analyten (110); und die zweite Erkennungskomponente (120) und ein Verstärkungselement (130), wobei die zweite Erkennungskomponente (120) optional mit dem Amplifikationselement (130) verbunden ist, wobei die zweite Erkennungskomponente (120), die optional mit dem Amplifikationselement (130) verbunden ist, Folgendes umfasst: die zweite Erkennungskomponente (120), die an einen ersten Bindungspartner (123) konjugiert ist, und das Amplifikationselement (130), das an einen zweiten Bindungspartner (135) konjugiert ist, der dafür konfiguriert ist, selektiv an den ersten Bindungspartner (123) zu binden, wobei bei Verwendung das Amplifikationselement (130) durch Bindung des ersten und des zweiten Bindungspartners (123 und 135) mit der zweiten Erkennungskomponente (120) verbunden ist;
einen Fluidweg von dem einen oder den mehreren Behältern (10) zur Oberfläche (26) des TFBAR (20), an den die erste Erkennungskomponente (100) gebunden ist;
eine Betätigungsschaltung (22), die dafür konfiguriert ist, den TFBAR (20) in einer oszillierenden Bewegung anzutreiben;
eine Messschaltung (29), die dafür ausgelegt ist, mit dem TFBAR (20) gekoppelt zu sein und dafür konfiguriert ist, ein oder mehrere Resonatorausgangssignale zu messen, die Resonanzeigenschaften der oszillierenden Bewegung des Erfassungsresonators darstellen; und
eine Steuerung (30), die betriebsfähig mit der Betätigungs- und Messschaltung (22 und 29) gekoppelt ist.

## Revendications

1. Procédé pour détecter un analyte dans un échantillon, comprenant :
l'entrée en contact avec un analyte (110) et optionnellement un analyte lié à étiquette, un premier composant de reconnaissance (100), et un second composant de reconnaissance (120) lié à un élément d'amplification (130) pour générer un complexe comprenant le premier composant de reconnaissance (100) et le second composant de reconnaissance (120) lié à l'élément d'amplification (130),
dans lequel le premier composant de reconnaissance (100) est immobilisé sur une surface (26) d'un résonateur acoustique de volume à film mince (TFBAR) (20) ayant une fréquence de résonance de 1,8 GHz ou plus et est configuré pour sélectivement associer un ou plusieurs de l'analyte (110) et optionnellement d'un analyte lié à étiquette associé au second composant de reconnaissance (120),
dans lequel le second composant de reconnaissance (120) lié à l'élément d'amplification (130) est configuré pour sélectivement associer l'analyte (110) et optionnellement l'analyte lié à étiquette associé au premier composant de reconnaissance (100) ; et
dans lequel le second composant de reconnaissance (120) est conjugué à un premier partenaire d'association (123), l'élément d'amplification (130) est conjugué à un second partenaire d'association (135) configuré pour s'associer sélectivement au premier partenaire d'association (123), et dans lequel l'élément d'amplification (130) est lié au second composant de reconnaissance (120) par le biais d'association entre les premier et second partenaires d'association (123 et 135) ;
l'entrée en contact avec l'élément d'amplification (130) lié à un précurseur d'amplification (140) dans des conditions pour convertir le précurseur d'amplification (140) en une molécule (150) qui ajoute une masse au niveau d'une surface (26) du TFBAR (20) ; et
la mesure de masse ajoutée au niveau de la surface (26) du TFBAR (20).

2. Procédé selon la revendication 1, dans lequel l'analyte (110) ou l'analyte et l'analyte lié à étiquette sont mis en contact avec le second composant de reconnaissance (120) lié à l'élément d'amplification (130) avant le contact avec le premier composant de reconnaissance (100) immobilisé sur la surface (26) du TFBAR (20).

3. Procédé selon la revendication 1, dans lequel l'analyte (110) ou l'analyte et l'analyte lié à étiquette sont mis en contact avec le premier composant de reconnaissance (100) avant le contact avec le second composant de reconnaissance (120) lié à l'élément d'amplification (130).

4. Procédé selon la revendication 1, dans lequel l'analyte (110) ou l'analyte lié à étiquette, le premier composant de reconnaissance (100) et le second composant de reconnaissance (120) lié à l'élément d'amplification (130) sont mis en contact simultanément.

5. Procédé selon l'une quelconque des revendications 1 à 4, dans lequel la mesure de la masse ajoutée à ou associée à la surface (26) du TFBAR (20) comprend :
le couplage d'un signal électrique d'entrée au TFBAR (20), le signal électrique d'entrée ayant une fréquence au sein d'une bande de résonance du résonateur piézoélectrique, dans lequel la fréquence est de 1,8 GHz ou plus ;
la transmission du signal électrique d'entrée à travers le TFBAR (20), ou par l'intermédiaire de ce dernier, pour générer un signal électrique de sortie ayant une fréquence ;
la réception du signal électrique de sortie à partir du TFBAR (20) ; et
la détermination d'un changement de déphasage du signal électrique de sortie causé par dépôt du précipité sur la surface (26) du TFBAR (20).

6. Procédé selon la revendication 5, dans lequel le changement de déphasage est un changement de déphasage de coefficient d'insertion ou de réflexion.

7. Procédé selon l'une quelconque des revendications 1 à 4, dans lequel la mesure de la masse ajoutée à ou associée à la surface (26) du TFBAR (20) comprend :
l'actionnement du TFBAR (20) en un mouvement oscillant à une fréquence d'environ 1,8 GHz ou plus ;
la mesure d'un ou de plusieurs signaux de sortie de résonateur représentant des caractéristiques de résonance du mouvement oscillant du TFBAR (20) ; et
l'ajustement de la fréquence d'actionnement du résonateur de détection pour maintenir un point de résonance du TFBAR (20).

8. Procédé selon la revendication 7, dans lequel le point de résonance du TFBAR (20) est un point de temps de propagation de groupe maximum.

9. Procédé selon l'une quelconque des revendications 5 à 8, dans lequel la fréquence est de 2 GHz ou plus.

10. Procédé selon l'une quelconque des revendications 5 à 8, dans lequel la fréquence est de 2 GHz à 2,5 GHz.

11. Procédé selon l'une quelconque des revendications 1 à 10, dans lequel l'élément d'amplification (130) est un enzyme et le précurseur d'amplification (140) est un substrat, et dans lequel l'enzyme est configuré pour convertir le substrat en un précipité.

12. Système pour détecter un analyte dans un échantillon, comprenant :
un résonateur acoustique de volume à film mince (TFBAR) (20) comprenant une surface (26) sur laquelle un premier composant de reconnaissance (100) est immobilisé,
le premier composant de reconnaissance (100) étant configuré pour sélectivement associer l'analyte (110) et optionnellement un analyte lié à étiquette auquel un second composant de reconnaissance (120) est associé, le TFBAR (20) ayant une fréquence de résonance de 1,8 GHz ou plus ;
un ou plusieurs contenants (10) logeant : un précurseur d'amplification (140) ; optionnellement un ou plusieurs de l'étiquette et de l'analyte (110) ; et le second composant de reconnaissance (120) et un élément d'amplification (130), le second composant de reconnaissance (120) étant optionnellement lié à l'élément d'amplification (130), dans lequel le second composant de reconnaissance (120) optionnellement lié à l'élément d'amplification (130) comprend le second composant de reconnaissance (120) conjugué à un premier partenaire d'association (123) et l'élément d'amplification (130) conjugué à un second partenaire d'association (135) qui est configuré pour sélectivement s'associer au premier partenaire d'association (123), dans lequel, durant l'utilisation, l'élément d'amplification (130) est lié au second composant de reconnaissance (120) par le biais d'association des premier et second partenaires d'association (123 et 135) ;
un chemin de fluide depuis les un ou plusieurs contenants (10) jusqu'à la surface (26) du TFBAR (20) auquel le premier composant de reconnaissance (100) est associé ;
une circuiterie d'actionnement (22) configurée pour entraîner le TFBAR (20) en un mouvement oscillant ;
une circuiterie de mesure (29) agencée pour être couplée au TFBAR (20) et configurée pour mesurer un ou plusieurs signaux de sortie de résonateur représentant des caractéristiques de résonance du mouvement oscillant du résonateur de détection ; et
une unité de commande (30) fonctionnellement couplée aux circuiteries d'actionnement et de mesure (22 et 29).
